# EUROPÄISCHE PATENTSCHRIFT

(11) **EP 0 846 685 B1**
(45) Veröffentlichungstag und Bekanntmachung des Hinweises auf die Patenterteilung: **13.03.2002**
(21) Anmeldenummer: 97120537.2
(22) Anmeldetag: 24.11.1997
(51) Int. Cl.: C07D 213/81, A61K 31/44

(54) **3-Hydroxy-Pyridin-2-carbonsäureamidester, ihre Herstellung und ihre Verwendung als Arzneimittel**
3-Hydroxypyridine-2-carboxylic acid amide esters, their preparation and their use as medicaments
Esters amides d'acide-2-carboxylique du 3-Hydroxpyridine, leur préparation et leur utilisation comme médicaments

(30) Priorität: 04.12.1996 DE 19650215
(43) Veröffentlichungstag der Anmeldung: 10.06.1998
(73) Patentinhaber: HOECHST AKTIENGESELLSCHAFT, 65929 Frankfurt am Main (DE)
(72) Erfinder: Weidmann, Klaus, Dr., 61476 Kronberg (DE); Baringhaus, Karl-Heinz, Dr., 61200 Wölfersheim (DE); Tschank, Georg, Dr., 55270 Klein-Winterheim (DE); Bickel, Martin, Dr., 61348 Bad Homburg (DE)

(56) Entgegenhaltungen:
- EP-A- 0 661 269
- EP-A- 0 765 871
- FRANKLIN T J ET AL: "Approaches to the design of anti-fibrotic drugs" BIOCHEM. SOC. TRANS. (BCSTB5,03005127);91; VOL.19 (4); PP.812-15, ICI PHARM.;MACCLESFIELD/CHESHIRE; SK10 4TG; UK (GB), XP002059028
- J.T.SHEEHAN: "3-Hydroxypicolinic acid and some of its derivatives" J ORG CHEM, Bd. 31, 1966, Seiten 636-638, XP002059029

## Beschreibung

Die Erfindung betrifft 3-Hydroxypyridin-2-carbonsäureamidester, ihre Herstellung und ihre Verwendung zur Hemmung der Kollagenbiosynthese und ihre Verwendung als Arzneimittel zur Behandlung von fibrotischen Erkrankungen.

Verbindungen, die die Enzyme Prolyl- und Lysylhydroxylase inhibieren, bewirken eine sehr selektive Hemmung der Kollagenbiosynthese durch Beeinflussung der kollagenspezifischen Hydroxylierungsreaktionen. In deren Verlauf wird proteingebundenes Prolin oder Lysin durch die Enzyme Prolyl- bzw. Lysylhydroxylase hydroxyliert. Wird diese Reaktion durch Inhibitoren unterbunden, so entsteht ein nicht funktionsfähiges, unterhydroxyliertes Kollagenmolekül, das von den Zellen nur in geringer Menge in den extrazellulären Raum abgegeben werden kann. Das unterhydroxylierte Kollagen kann außerdem nicht in die Kollagenmatrix eingebaut werden und wird sehr leicht proteolytisch abgebaut. Als Folge dieser Effekte verringert sich insgesamt die Menge des extrazellulär abgelagerten Kollagens.

Inhibitoren der Prolylhydroxylase sind deshalb geeignete Substanzen in der Therapie von Erkrankungen, in denen die Ablagerung von Kollagenen maßgeblich zum Krankheitsbild beiträgt. Hierzu gehören u. a. Fibrosen der Lunge, Leber und Haut (Skleroderma und Vernarbungen nach Verbrennungen, Verletzungen und chirurgischen Eingriffen) sowie die Artherosklerose.

Es ist bekannt, daß das Enzym Prolylhydroxylase durch Pyridin-2,4- und -2,5-dicarbonsäure effektiv gehemmt wird (K. Majamaa et al., Eur. J. Biochem. 138 (1984) 239-245). Diese Verbindungen sind in der Zellkultur allerdings nur in sehr hohen Konzentrationen als Hemmstoffe wirksam (Tschank, G. et al., Biochem. J. 238 (1987) 625-633).
Auch Prodrugs der Pyridin-2,4(5)-dicarboxylate sind bekannt. Diese sind in den Patentanmeldungen EP-A-0 590 520 und DE-A-42 38 506 und EP-A-0 562 512 beschrieben.

N-Oxalylglycine als Inhibitoren der Prolyl-4-hydroxylase sind aus J.Med.Chem. 1992, 35, 2652-2658 (Cunliffe et al.) und EP-A-0 457 163 bekannt.

3-Hydroxypyridin-2-carbonsäure(glycyl)amide und deren Prodrugs sowie deren Verwendung sind aus der EP-A-0 661 269 bekannt.

Weiterhin ist 3-Hydroxypyridin-2-carbonsäure(glycylethylester)amid in J. Org. Chem. 31, 636-638 (1966) beschrieben.

Es bestand daher die Aufgabe, Verbindungen bereitzustellen, die sich durch eine besonders hohe In-vivo und/oder In-vitro-Aktivität auszeichnen, insbesondere bei ihrer systemischen und/oder lokalen Anwendung.

Überraschenderweise wurde nun gefunden, daß 3-Hydroxypyridin-2-carbonsäureamidester der Formel I besonders stark wirksame Inhibitoren der Kollagenbiosynthese sind. Sie zeichnen sich durch eine besonders hohe In-vivo- und In-vitro-Aktivität aus, insbesondere bei ihrer systemischen und/oder lokalen Verwendung gegen fibrotische Erkrankungen. Hierzu gehören beispielsweise Fibrosen der Lunge, der Leber, der Niere, des Herzens, des Auges sowie der Haut und bei Artherosklerose.

Die Verbindungen der Formel I führen zu einer starken Hemmung der Kollagenbiosynthese in den verschiedenartigsten Zellen (z.B. normale humane Hautfibroblasten, primäre Fettspeicherzellen aus der Rattenleber, Rattenleberepithelzellen und in Organkulturen von Calvarien).

Sie sind besonders geeignet zur lokalen Anwendung als Inhibitoren der Kollagenbiosynthese, zur lokalen Anwendung als antifibrotische Wirkstoffe und zur lokalen Anwendung bei Krankheitsformen, die von einer erhöhten Bildung von Bindegewebe (Kollagen) verursacht werden. So sind die Verbindungen der Formel I z.B. geeignet zur lokalen Anwendung zur Vermeidung/Verringerung von Vernarbungen nach chirurgischen Eingriffen am menschlichen Körper und zur lokalen Anwendung bei der postoperativen Behandlung von Augenerkrankungen, z.B. der nachoperativen Behandlung des Glaukoms und bei strahleninduzierter oder durch Chemotherapie induzierter Fibrose, insbesondere an der Lunge.

Die erfindungsgemäßen Verbindungen sind Ester-Prodrugs der entsprechenden Carbonsäuren der Formel I, in denen B eine Carboxylgruppe bedeutet.

Die Verbindungen der Formel I werden im lebenden Organismus (in vivo) und in Zellkulturen (in vitro) zu Verbindungen der Formel I, in denen B eine Carboxylgruppe oder deren Salze bedeutet, gespalten.

Nach der Applikation der Verbindungen der Formel I bewirken sie die in vivo und in vitro zu beobachtende Hemmung der Kollagenbiosynthese, wobei sich die Verbindungen der Formel I, in denen B eine Carboxylgruppe oder deren Salze bedeutet, bilden. Diese Verbindungen inhibieren die Prolyl-4-hydroxylase und führen deshalb zu einer Hemmung der Kollagenbiosynthese.

Die erfindungsgemäßen Verbindungen entsprechen der allgemeinen Formel I 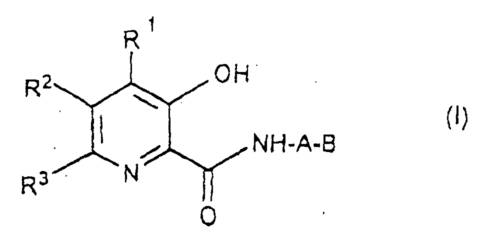 in welcher
- R¹, R² und R³: Wasserstoff,
- A: eine -CH₂-Gruppe bedeutet, in der ein Wasserstoff durch eine Methylgruppe ersetzt sein kann, und
- B: -CO₂G bedeutet, wobei
- G: einen verzweigten oder unverzweigten (C₁-C₂₀)-Alkyl-Rest, einen verzweigten oder unverzweigten (C₂-C₂₀)-Alkenyl-Rest, einen entsprechenden (C₂-C₂₀)-Alkinyl-Rest, einen entsprechenden (C₄-C₂₀)-Alkeninyl-Rest oder einen (C₃-C₁₂)-Cycloalkylrest, oder einen Retinyl-Rest bedeutet, wobei die Reste jeweils eine oder mehrere Mehrfachbindungen enthalten können, oder einen Phenylalkyl-Rest bedeutet, wobei die vorstehenden Reste insbesondere einen oder mehrere Substituenten aus der Reihe Hydroxy, Halogen, Cyano, Trifluormethyl, Carboxy, (C₁-C₁₂)-Alkyl, (C₃-C₈)-Cycloalkyl, (C₅-C₈)-Cycloalkenyl, (C₁-C₁₂)-Alkoxy, (C₁-₁₂)-Alkoxy-(C₁-C₁₂)-alkyl, (C₁-C₁₂)-Alkoxy-(C₁-C₁₂)-alkoxy, Phenyl-(C₁-C₄)-alkyloxy, (C₁-C₈)-Hydroxyalkyl, (C₁-C₁₂)-Alkylcarbonyloxy, (C₃-C₈)-Cycloalkylcarbonyloxy, Benzoyloxy, Phenyl-(C₁-C₄)-alkylcarbonyloxy enthalten, einschließlich der physiologisch wirksamen Salze, ausgenommen 3-Hydroxypyridin-2-carbonsäure-(glycylethylester)amid.

Bevorzugt sind Verbindungen der Formel I, in denen
- R¹, R² und R³: Wasserstoff,
- A: eine -CH₂-Gruppe bedeutet, in der ein Wasserstoffatom durch eine Methylgruppe ersetzt sein kann, und
- B: -CO₂G bedeutet, wobei
- G: einen verzweigten oder unverzweigten aliphatischen (C₁-C₁₈)-Alkyl-Rest, einen (C₃-C₈)-Cycloalkylrest, einen (C₃-C₈)-Cycloalkyl-(C₁-C₈)-alkylrest, einen verzweigten oder unverzweigten (C₂-C₁₈)-Alkenylrest; wie z.B. einen Geranyl-, Farnesyl- oder einen Retinyl-Rest, oder einen entsprechenden (C₂-C₁₈)-Alkinylrest, einen Benzyl-, Phenethyl-, Phenylpropyl- oder Phenylbutylrest bedeutet,
wobei die vorstehenden Reste einen Substituenten aus der Reihe Hydroxy, (C₁-C₄)-Alkoxy, (C₁-C₆)-Alkylcarbonyloxy, (C₃-C₈)-Cycloalkylcarbonyloxy, Benzoyloxy oder Phenyl-(C₁-C₄)-alkylcarbonyloxy, enthalten, einschließlich der physiologisch wirksamen Salze, ausgenommen 3-Hydroxypyridin-2-carbonsäure-(glycylethylester)amid.

Besonders bevorzugt sind Verbindungen der Formel I, in denen
- R¹, R² und R³: Wasserstoff,
- A: eine -CH₂-Gruppe bedeutet, in der ein Wasserstoffatom durch eine Methylgruppe ersetzt sein kann, und
- B: -CO₂G bedeutet, wobei
- G: einen verzweigten oder unverzweigten aliphatischen (C₁-C₁₈)-Alkyl-Rest, einen (C₅-C₆)-Cycloalkylrest, einen (C₃-C₈)-Cycloalkyl-(C₁-C₄)-alkylrest, einen verzweigten oder unverzweigten (C₂-C₁₈)-Alkenylrest, einen Benzyl-, Phenethyl-, Phenylpropyl- oder Phenylbutylrest, bedeutet, einschließlich der physiologisch wirksamen Salze, ausgenommen 3-Hydroxypyridin-2-carbonsäure-(glycylethylester)amid.

Besonders bevorzugt sind weiterhin Verbindungen der Formel I, in denen
- R¹, R² und R³: Wasserstoff,
- A: eine -CH₂-Gruppe bedeutet, in der ein Wasserstoff durch eine Methylgruppe ersetzt sein kann, und
- B: -CO₂G bedeutet, wobei
- G: einen verzweigten oder unverzweigten (C₁-C₁₈)-Alkyl-oder (C₂-C₁₈)-Alkenyl-Rest, bedeutet, einschließlich der physiologischen wirksamen Salze, ausgenommen 3-Hydroxypyridin-2-carbonsäure-(glycylethylester)amid.

Besonders bevorzugt sind weiterhin Verbindungen der Formel I, in denen
- R¹, R² und R³: Wasserstoff,
- A: eine -CH₂-Gruppe, und
- B: -CO₂G bedeutet, wobei
- G: einen verzweigten oder unverzweigten (C₁-C₁₈)-Alkyl- oder (C₂-C₁₈)-Alkenyl-Rest bedeutet,
einschließlich der physiologisch wirksamen Salze, ausgenommen 3-Hydroxypyridin-2-carbonsäure-(glycylethylester)amid.

Insbesondere bevorzugt sind Verbindungen der Formel I, in denen
- R¹, R² und R³: Wasserstoff,
- A: eine -CH₂-Gruppe,
- B: -CO₂G bedeutet, wobei
- G: einen linearen (C₁-C₁₈)-Alkyl-Rest bedeutet, einschließlich der physiologisch wirksamen Salze, ausgenommen 3-Hydroxypyridin-2-carbonsäure-(glycylethylester)amid.

Besonders bevorzugt sind solche Verbindungen der Formel I, in denen
- G: einen linearen (C₁-C₁₆)-Alkyl-Rest bedeutet, einschließlich der physiologisch wirksamen Salze, ausgenommen 3-Hydroxypyridin-2-carbonsäure-(glycylethylester)amid.

Die Erfindung umfaßt weiterhin Salze der Verbindungen der allgemeinen Formel I.

Die Salzbildung mit sauren Reagenzien kann am Pyridin-N-Atom erfolgen.

Zur Anwendung kommende Reagenzien sind beispielsweise Toluolsulfonsäure, Methansulfonsäure, HCI, H₂SO₄, H₃PO₄ und Arzneimittel, die eine saure Gruppe enthalten.

Die Erfindung betrifft die Verbindungen der allgemeinen Formel I sowie die physiologisch verträglichen Salze zur Anwendung bei der Hemmung der Kollagenbiosynthese.

Die Erfindung betrifft die Verbindungen der allgemeinen Formel I sowie die physiologisch verträglichen Salze zur Anwendung bei der Hemmung der Prolyl-4-hydroxylase in vivo und in vitro.

Weiterhin betrifft die Erfindung die Verbindungen der allgemeinen Formel I sowie die physiologisch verträglichen Salze zur Anwendung bei fibrotischen Erkrankungen der Lunge, der Leber, der Niere, des Herzens, des Auges und der Haut. Die Verbindungen können auch Anwendung finden bei Artherosklerose. Hierbei werden systemische und/oder lokale Applikationen angewandt.

Insbesondere betrifft die Erfindung Verbindungen der allgemeinen Formel I sowie der physiologisch verträglichen Salze zur lokalen Anwendung, insbesondere als Inhibitoren der Kollagenbiosynthese, als antifibrotische Wirkstoffe und bei Krankheitsformen, die von einer erhöhten Bildung von Bindegewebe (Kollagen) verursacht werden. Hierunter zählen die Anwendungen zur Vermeidung/Verringerung von Vernarbungen nach chirurgischen Eingriffen am menschlichen Körper und bei der postoperativen Behandlung von Augenoperationen, z.B. bei Glaukomoperationen und bei strahleninduzierter oder durch Chemotherapie induzierter Fibrose, insbesondere an der Lunge.

Schließlich betrifft die Erfindung die Verbindungen der allgemeinen Formel I zur Verwendung als Arzneimittel.

Insbesondere betrifft die Erfindung Verbindungen der Formel I zur lokalen Anwendung bei Fibrosen der Haut, der Lunge und des Auges, insbesondere zur nachoperativen Behandlung des Glaukoms.

Weiterhin betrifft die Erfindung ein Verfahren zur Herstellung von Verbindungen der allgemeinen Formel I.

Die Herstellung der Verbindungen der Formel I, in denen A eine -CH₂-Gruppe, in der ein Wasserstoff durch eine Methylgruppe ersetzt sein kann und B CO₂G bedeuten, erfolgt, indem
i1.) Pyridin-2-carbonsäuren der Formel II (R⁵ = H) mit den Aminoestern Formel III oder deren Salze zu den Amidestern der Formel I umgesetzt werden; oder
i2.) Pyridin-2-carbonsäureester der Formel II (R⁵ = niedrig Alkyl (=C₁-C₅)-Alkyl) unter den Bedingungen der Aminolyse zu den Verbindungen der Formel I umgesetzt werden; oder
ii) die Verbindungen der Formel IV mit einem Alkohol GOH verestert werden.

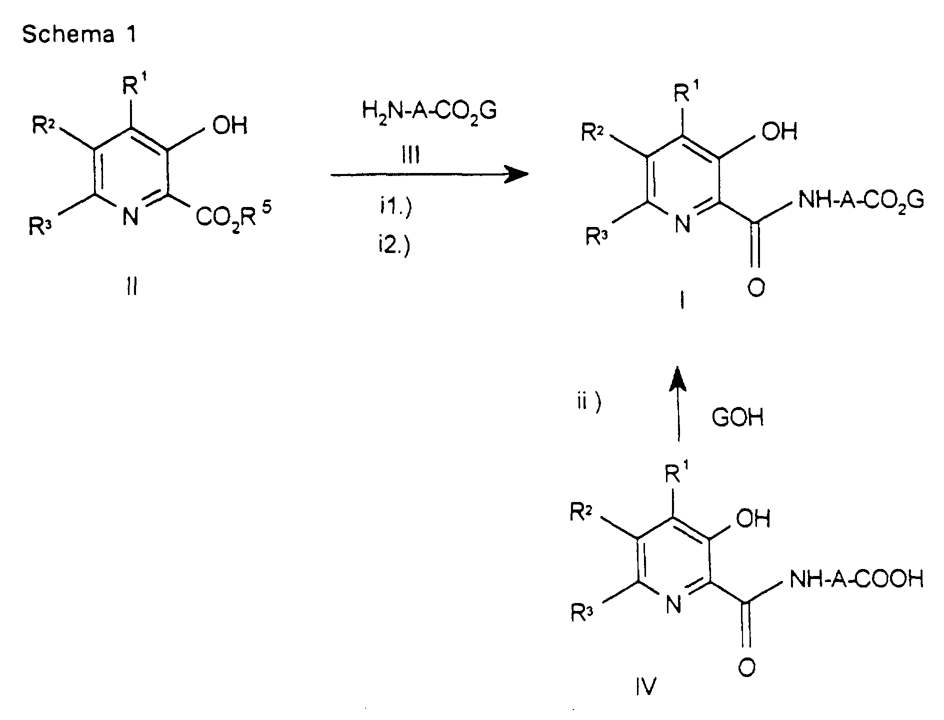

Geeignete Verfahren zur Amidbildung (Umsetzung i1) sind die Methoden der Carbonylaktivierung und die aus der Peptidchemie bekannten Kondensationsreaktionen.

An Reagenzien zur Carbonsäureaktivierung können die dem Fachmann bekannten Substanzen, wie Thionylchlorid, Oxalylchlorid, Pivaloylchlorid, Chlorameisensäureester-Derivate oder N,N'-Carbonyldimidazol Verwendung finden. Die aktivierten Derivate der Verbindungen der Formel II werden nach Herstellung in situ mit den Amidderivaten der Formel III umgesetzt.

Ein geeignetes Kondensationsmittel ist beispielsweise die Kombination von N,N'-Dicyclohexylcarbodiimid/N-Hydroxy-1H-benzotriazol und N-Ethylmorpholin.

Geeignete Lösungsmittel sind Dichlormethan, Tetrachlormethan, Butylacetat, Ethylacetat, Toluol, Tetrahydrofuran, Dimethoxyethan, 1,4-Dioxan, Acetonitril, N,N-Dimethylformamid, N,N-Dimethylacetamid, Dimethylsulfoxid, Nitromethan und/oder Pyridin.

Die erfindungsgemäßen Verbindungen der Formel I besitzen wertvolle pharmakologische Eigenschaften und zeigen insbesondere antifibrotische Wirksamkeit.

Die antifibrotische Wirkung kann in folgenden Tiermodellen bestimmt werden:
Bildung einer bindegewebigen Kapsel um eine subkutan implantierte osmotische Minipumpe, in: Unemori, E.N. et al., J. Invest. Dermatol., 101:280-5, 1993,
Kollagengehalt von subkutan implantierten "cotton pellets" oder Polyvinylschwämmchen, in: Boyle, E., Mangan, F.R., Br. J. Ep. Pathnol. 61: 351-60, 1980; und
Kollagengehalt der Lunge nach strahleninduzierter oder chemisch induzierter Fibrose, Radiat. in: Ward, H.E. et al., Res., 136, 15-21, 1993, sowie Santana, A. et al. Am. J. Respir. Cell. Mol. Biol. 13: 34-44, 1995.

Hemmung der Prolyl-4-hydroxylase in Zellkulturen: Normale humane Hautfibroblasten (Normal human fibroblasts, NHDF), Rattenleber-Epithelzellen (rat liver epithelial cells, Ref. 1) und Fettspeicherzellen aus der Leber (fat storing cells, Ref. 2) werden als Zellmodelle zur Substanztestung verwendet. Dazu werden die Zellen in Gegenwart von Hemmstoffen kultiviert. Gleichzeitig wird das in dieser Zeit neu synthetisierte Kollagen durch 4-³H-L-Prolin und ¹⁴C-Prolin metabolisch markiert. Der Einfluß der Testsubstanzen auf den Hydroxylierungsgrad des Kollagens wird anschließend entsprechend der Methode von Chojkier et al (Ref. 3) bestimmt. Als Referenzsubstanz wird 2,2'-Dipyridyl eingesetzt. (1.: Schrode, W., Mecke, D., Gebhard, R. 1990. Induction of glutamine synthetase in periportal hepatocytes by co-cultivation with a liver epithelial cell line. Eur. J. Cell. Biol. 53: 35-41, 2.: Blomhoff, R., Berg T. 1990. Isolation and cultivation of rat liver stellate cells. Methods Enzymol. 190: 59-71 und 3.: Chojkier, M. Peterkofsky, B. Bateman,, J. 1980. A new method for determining the extent of proline hydroxylation by measuring changes in the ration of [4-³H]:[¹⁴C] proline in collagenase digests. Anal. Biochem. 108: 385-393).

Hemmung der Prolyl-4-hydroxylase in vitro (in Hühnchenembryo - Calvarien) gemäß Biochem. J. (1994) 300, 525-300:

### 1. Metabolische Markierung

Calvarien werden aus 15 Tage alten Hühnchenembryonen präpariert und 3 min bei 37°C in Hanks' balanced salt solution minimum essential medium Eagle (HMEM, BioWhittaker, Walkersville, MD, USA) gewaschen. Je 4 Calvarien werden in Glasgefäßen mit 1.5 ml HMEM unter Zusatz von 2 mM Glutamin und 1 µCi [U-14C]Prolin sowie verschiedenen Inhibitorkonzentrationen 2.5 h bei 37°C inkubiert. Die Inkubation wird durch Einbringen der Probengefäße in Eis beendet. Das Medium wird entfernt und die Calvarien werden kurz mit 1 ml bidest H₂O gewaschen. Danach werden die Calvarien mit 3 ml 0.5 M Essigsäure und 18 µg Phenylmethan-Sulfonylfluorid versetzt und 16 h extrahiert. Die Extraktionslösung wird bis zum Entfernen des freien [U-14C]Prolin bei 4°C gegen 0.5 M Essigsäure dialysiert und anschließend in Aliquots gefriergetrocknet.

### 2. Hydroxyprolin - Analyse

Ein Aliquot der gefriergetrockneten Probe wird in 2 ml 6 N HCI aufgelöst und 24 h bei 105°C hydrolysiert. Danach wird die Salzsäure abgedampft. Der Rückstand wird in 300 µl bidest H₂O resuspendiert, in Eppendorf-Gefäße überführt und erneut getrocknet. Die verbleibenden Hydrochloride werden durch Lösen in 60 µl einer Ethanol/H₂O/Triethylamin 2:2:1 (v:v:v) und erneutem Trocknen neutralisiert. Anschließend werden die Aminosäuren 20 min bei Raumtemperatur in einer Lösung von Ethanol/Triethylamin/Phenylisothiocyanat/H20 7:1:1:1 (v:v:v:v) derivatisiert und erneut getrocknet. Zur Analyse mit HPLC werden die Proben in 150 µl eines Phosphatpuffers (5 mM Na₂HPO₄, pH 7.4/Acetonitril 95:5 (v:v)) gelöst und 5 min bei 10000 g zentrifugiert. 50 µl werden zur Analyse verwendet. Die HPLC-Chromatographie erfolgt auf einer C18 reverse phase Säule Ultrasphere ODS 3 µm, 4.6 nim x 7.5 cm (Beckman) bei 50°C mit folgendem Gradientensystem:

| Zeit | Puffer A | Puffer B |
|---|---|---|
| 0 min | 100% | |
| 0-9 min | 100-90 % | 0-10 % |
| 9-11 min | 90-0 % | 10-100 % |
| 11-12 min | 0% | 100% |
| 12-14 min | 0-100 % | 100-0 % |
| 14-19 min | 100% | |

Lsg A: 70 mM Na- Acetat pH 6.14/0.1 % Acetonitril
Lsg. B: Acetonitril/Methanol/Wasser 45:15:40 (v:v:v)

### 3. SDS - Polyacrylamid - Gelelektrophorese

Ein Aliquot der gefriergetrockneten Proben aus 1. wird für die SDS-Polyacrylamid - Gelelektrophorese mit 10 mM Tris/HCl, pH 8 1 mM EDTA / 1 % SDS und 5 % Mercaptoethanol gelöst und 5 min bei 95°C denaturiert. Die Elektrophorese erfolgt auf einem linearen Gradientengel (5-15 %). Nach Fixierung in Methanol/Eisessig/Wasser 3:1:6 (v:v:v) werden die Gele getrocknet und zur Belichtung von Kodak X-Omat Film bei -70°C verwendet.

Im folgenden werden die überraschenden Vorteile der erfindungsgemäßen Verbindungen gegenüber Verbindungen aus EP-A-0 661 269 anhand von Vergleichsversuchen über die Hemmung der Prolyl-4-hydroxylase (Kollagenbiosynthese) in Rattenleberzellen verdeutlicht:

**Tabelle 1:**

| Verbindungen aus Beispiel-Nr. | Hemmung der Kollagenbiosynthese in Rattenleberzellen |
|---|---|
| der EP-A-0 661 269 | Rattenleberfettspeicher (Ito) Zellen |
| 1 | -50 % : 53 µM |
| der EP-A-0 661 269 | Rattenleberepithelzellen |
| 1 | -50 % : 38 µM |

**Tabelle 2:**

| Verbindungen aus Beispiel-Nr. | Hemmung der Kollagenbiosynthese in Zellen |
|---|---|
| der vorliegenden Anmeldung | Rattenleberepithelzellen |
| 1 | -100 % : 25 µM |
| der vorliegenden Anmeldung | normale humane Hautfibroblasten (NHDF) |
| 1 | -50 % : 0,6 µM |
| 2 | -50 % : 1,6 µM |

**Tabelle 3:**

| Verbindungen aus Beispiel-Nr. der vorliegenden Anmeldung | in Calvarien |
|---|---|
| 19 | -50 % : < 0,8 µM |
| 20 (Disclaimer) | -50 % : < 0,8 µM |

Die Verbindungen der Formel I können als Medikamente in Form pharmazeutischer Präparate Verwendung finden, welche sie gegebenenfalls mit verträglichen pharmazeutischen Trägern enthalten. Die Verbindungen können als Heilmittel, z.B. in Form pharmazeutischer Präparate Verwendung finden, welche diese Verbindungen in Mischung mit einem für die enterale, perkutane, parenterale und inhalative Applikation geeigneten pharmazeutischen, organischen oder anorganischen Träger, wie z.B. Wasser, Gummi arabicum, Gelatine, Milchzucker, Stärke, Magnesiumstearat, Talk, pflanzliche Öle, Polyalkylenglykole, Vaseline usw. enthalten.

Sie können zu diesem Zweck oral in Dosen von 0,1 bis 25 mg/kg/Tag, vorzugsweise 1 bis 5 mg/kg/Tag oder parenteral in Dosen von 0,01 bis 5 mg/kg/Tag, vorzugsweise 0,01 bis 2,5 mg/kg/Tag, insbesondere 0,5 bis 1,0 mg/kg/Tag, appliziert werden. Die Dosierung kann in schweren Fällen auch erhöht werden. In vielen Fällen genügen jedoch auch geringere Dosen. Diese Angaben beziehen sich auf einen Erwachsenen von etwa 75 kg Gewicht.

Unter den im folgenden beschriebenen Beispielen werden die erfindungsgemäßen Verbindungen der Formel I als substituierte 3-Hydroxypyridin-2-carbonsäure(glycylester)amide bezeichnet.
Unter dieser Bezeichnungsweise werden substituierte 3-Hydroxypyridin-2-carbonsäure-N-((alkoxycarbonyl)methyl)amide verstanden.
Die Klassifizierung als substituierte N-(3-Hydroxypyridyl-2-carbonyl)glycinester ist eine weitere Möglichkeit.

### Beispiel 1

### 3-Hydroxypyridin-2-carbonsäure-N-(((1-dodecyloxy)carbonyl)methyl)amid

a) Glycin-1-dodecylester-Tosylat
   38 g (0,5 Mol) Glycin, 93,2 g (0,5 Mol) 1-Dodekanol und 115 g (0,6 Mol) p-Toluolsulfonsäure wurden mit 400 ml Toluol am Wasserabscheider erhitzt bis kein Wasser mehr überging. 4 h nach dem Abkühlen wurde durch Anreiben zur Kristallisation gebracht, mit Diethylether verdünnt, das ausgefallene Produkt abgesaugt, mit Diethylether gewaschen und getrocknet. Man erhielt 140 g Produkt, Fp. 106°C (sintern bei 80°C).
b) 7 g (50 mMol) 3-Hydroxypyridin-2-carbonsäure wurden in 800 ml wasserfreiem Dichlormethan suspendiert und mit 7,5 g (55 mMol) 1-Hydroxy-1H-benztriazol, 19,2 ml (150 mMol) N-Ethylmorpholin, 21,2 g (50 mMol) N-Cyclohexyl-N'-(2-morpholinoethyl)carbodiimid-methyl-p-toluolsulfonat (CMC) und 20,8 g (50 mMol) Glycin-1-dodecylester-Tosylat versetzt und 50 h bei 20°C gerührt.

Dann wurde der ausgefallene Harnstoff abgesaugt, die Mutterlauge nacheinander mit 2N wäßriger Salzsäure, mit gesättigter, wäßriger Nabicarbonat-Lösung und mit Wasser geschüttelt, die organische Phase getrocknet, i.Vak. eingeengt und der Rückstand mit Ethylacetat an Kieselgel chromatographiert. Aus entsprechenden Fraktionen wurden 12 g hellbraunes Öl erhalten, das beim Stehen kristallisierte. Man behandelte mit kaltem Petrolether, saugte das Produkt ab, wusch mit kaltem Petrolether und erhielt 8,5 g farblos kristallines Produkt, Fp. 53-56°C.

### Beispiel 2

### 3-Hydroxypyridin-2-carbonsäure-N-(((1-butyloxy)carbonyl)methyl)amid

a) Glycin-1-butylester-Tosylat
   38 g (0,5 Mol) Glycin wurden in 400 ml Toluol mit 37 g (0,5 Mol) 1-Butanol und 115 g (0,6 Mol) p-Toluolsulfonsäure versetzt und am Wasserabscheider erhitzt bis kein Wasser mehr überging. Nach Stehen über Nacht wurde das ausgefallene Produkt abgesaugt und mit Diethylether gewaschen; 20 g Produkt, Fp.: 80-82°C. Aus der Mutterlauge wurden weitere 130 g Produkt gewonnen.
b) Die Titelverbindung wurde erhalten, indem 4,9 g (35 mMol) 3-Hydroxypyridin-4-carbonsäure analog zu Beispiel 1b) mit 4,7 g (35 mMol) 1-Hydroxy-1H-benztriazol, 19,8 ml (150 mMol) N-Ethylmorpholin, 14,8 g (35 mMol) CMC und 10,6 g Glycin-1-butylester-Tosylat umgesetzt wurde. Nach Chromatographie des Rohprodukts mit Ethylacetat an Kieselgel wurden 3 g öliges, hellbraunes Produkt erhalten.

¹H-NMR (DMSO, 300 MHz): δ = 12,21 (s, OH), 9,45 (t, NH), 8.20 (m, 1H), 7,55 (m, 1H), 7.43 (m, 1H), 4,12 (d, CH₂), 4,08 (OCH₂), 1.56 (m, 2H), 1,33 (m, 2H), 0,88 (t, CH₃).

Beispiel 3

3-Hydroxypyridin-2-carbonsäure-N-(((1-octyloxy)carbonyl)methyl)amid

Beispiel 4

3-Hydroxypyridin-2-carbonsäure-N-(((1-pentyloxy)carbonyl)methyl)amid

Beispiel 5

3-Hydroxypyridin-2-carbonsäure-N-(((1-hexyloxy)carbonyl)methyl)amid

Beispiel 6

3-Hydroxypyridin-2-carbonsäure-N-(((1-heptyloxy)carbonyl)methyl)amid

Beispiel 7

3-Hydroxypyridin-2-carbonsäure-N-(((1-nonyloxy)carbonyl)methyl)amid

Beispiel 8

3-Hydroxypyridin-2-carbonsäure-N-(((1-decyloxy)carbonyl)methyl)amid

Beispiel 9

3-Hydroxypyridin-2-carbonsäure-N-(((1-tetradecyloxy)carbonyl)methyl)amid

Beispiel 10

3-Hydroxypyridin-2-carbonsäure-N-(((1-octadecyloxy)carbonyl)methyl)amid

Beispiel 11

3-Hydroxypyridin-2-carbonsäure-N-[(((2-ethyl)-1-butyloxy)carbonyl)methylamid Ölige Substanz; MS: m/e = 281,3 (M⁺ +H)

Beispiel 12

3-Hydroxypyridin-2-carbonsäure-N-(((1-tridecyloxy)carbonyl)methyl)amid

Beispiel 13

3-Hydroxypyridin-2-carbonsäure-N-(((1-hexadecyloxy)carbonyl)methyl)amid

Beispiel 14

3-Hydroxypyridin-2-carbonsäure-N-((1-(cis-9-octadecenyl)oxy)carbonyl)methyl)amid

Beispiel 15

3-Hydroxypyridin-2-carbonsäure-N-(((1-(trans-3-hexenyl)oxy)carbonyl)methyl)amid

Beispiel 16

3-Hydroxypyridin-2-carbonsäure-N-(((1-(3-methylbutyl)oxy)carbonyl)methyl)amid

Beispiel 17

3-Hydroxypyridin-2-carbonsäure-N-(((1-propyloxy)carbonyl)methyl)amid

Beispiel 18

3-Hydroxypyridin-2-carbonsäure-N-(((2-propyloxy)carbonyl)methyl)amid

Beispiel 19

3-Hydroxypyridin-2-carbonsäure-N-(((3-pentyloxy)carbonyl)methyl)amid, Fp. 84°C (aus n-Heptan/Ethylacetat (5:1))

Beispiel 20

3-Hydroxypyridin-2-carbonsäure(glycylethylester)amid (J. Org. Chem. 31, 636-638 (1966)).

## Patentansprüche

1. Verbindungen der allgemeinen Formel I 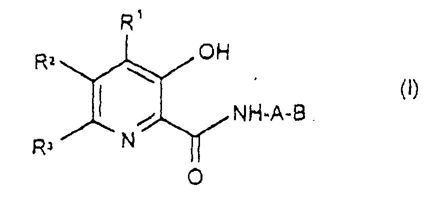 in welcher
R¹, R² und R³ Wasserstoff,
A eine -CH₂-Gruppe bedeutet, in der ein Wasserstoff durch eine Methylgruppe ersetzt sein kann, und
B -CO₂G bedeutet, wobei
G einen verzweigten oder unverzweigten (C₁-C₂₀)-Alkyl-Rest, oder einen verzweigten oder unverzweigten (C₂-C₂₀)-Alkenyl-Rest, einen entsprechenden (C₂-C₂₀)-Alkinyl-Rest, einen entsprechenden (C₄-C₂₀)-Alkeninyl-Rest oder einen (C₃-C₁₂)-Cycloalkylrest, oder einen Retinyl-Rest bedeutet, wobei die Reste jeweils eine oder mehrere Mehrfachbindungen enthalten können, oder einen Phenylalkyl-Rest bedeutet, wobei die vorstehenden Reste insbesondere einen oder mehrere Substituenten aus der Reihe Hydroxy, Halogen, Cyano, Trifluormethyl, Carboxy, (C₁-C₁₂)-Alkyl, (C₃-C₈)-Cycloalkyl, (C₅-C₈)-Cycloalkenyl, (C₁-C₁₂)-Alkoxy, (C₁-C₁₂)-Alkoxy-(C₁-C₁₂)-alkyl, (C₁-C₁₂)-Alkoxy-(C₁-C₁₂)-alkoxy, Phenyl-(C₁-C₄)-alkyloxy, (C₁-C₈)-Hydroxyalkyl, (C₁-C₁₂)-Alkylcarbonyloxy, (C₃-C₈)-Cycloalkylcarbonyloxy, Benzoyloxy, Phenyl-(C₁-C₄)-alkylcarbonyloxy enthalten, einschließlich der physiologisch wirksamen Salze, ausgenommen 3-Hydroxypyridin-2-carbonsäure-(glycylethylester)-amid,

2. Verbindungen der Formel I gemäß Anspruch 1, in denen
R¹, R² und R³ Wasserstoff,
A eine -CH₂-Gruppe bedeutet, in der ein Wasserstoffatom durch eine Methylgruppe ersetzt sein kann, und
B -CO₂G bedeutet, wobei
G einen verzweigten oder unverzweigten aliphatischen (C₁-C₁₈)-Alkyl-Rest, einen (C₃-C₈)-Cycloalkylrest, einen (C₃-C₈)-Cycloalkyl-(C₁-C₈)-alkylrest, einen verzweigten oder unverzweigten (C₂-C₁₈)-Alkenylrest, wie z.B. einen Geranyl-, Farnesyl- oder einen Retinyl-Rest, oder einen entsprechenden (C₂-C₁₈)-Alkinylrest, einen Benzyl-, Phenethyl-, Phenylpropyl- oder Phenylbutylrest bedeutet,
wobei die vorstehenden Reste einen Substituenten aus der Reihe Hydroxy, (C₁-C₄)-Alkoxy, (C₁-C₆)-Alkylcarbonyloxy, (C₃-C₈)-Cycloalkylcarbonyloxy, Benzoyloxy oder Phenyl-(C₁-C₄)-alkylcarbonyloxy, enthalten, einschließlich der physiologisch wirksamen Salze, ausgenommen 3-Hydroxypyridin-2-carbonsäure-(glycylethylester)-amid.

3. Verbindungen der Formel I gemäß den Ansprüchen 1 bis 2, in denen
R¹, R² und R³ Wasserstoff,
A eine -CH₂-Gruppe bedeutet, in der ein Wasserstoffatom durch eine Methylgruppe ersetzt sein kann, und
B -CO₂G bedeutet, wobei
G einen verzweigten oder unverzweigten aliphatischen (C₁-C₁₈)-Alkyl-Rest, einen (C₅-C₆)-Cycloalkylrest, einen (C₃-C₈)-Cycloalkyl-(C₁-C₄)-alkylrest, einen verzweigten oder unverzweigten (C₂-C₁₈)-Alkenylrest, einen Benzyl-, Phenethyl-, Phenylpropyl- oder Phenylbutylrest, bedeutet, einschließlich der physiologisch wirksamen Salze, ausgenommen 3-Hydroxypyridin-2-carbonsäure-(glycylethylester)-amid.

4. Verbindungen der Formel I gemäß den Ansprüchen 1 bis 3, in denen
R¹, R² und R³ Wasserstoff,
A eine -CH₂-Gruppe bedeutet, in der ein Wasserstoff durch eine Methylgruppe ersetzt sein kann, und
B -CO₂G bedeutet, wobei
G einen verzweigten oder unverzweigten (C₁-C₁₈)-Alkyl-oder (C₂-C₁₈)-Alkenyl-Rest, bedeutet, einschließlich der physiologischen wirksamen Salze, ausgenommen 3-Hydroxypyridin-2-carbonsäure-(glycylethylester)-amid.

5. Verbindungen der Formel I gemäß den Ansprüchen 1 bis 4, in denen
R¹, R² und R³ Wasserstoff.
A eine -CH₂-Gruppe, und
B -CO₂G bedeutet, wobei
G einen verzweigten oder unverzweigten (C₁-C₁₈)-Alkyl- oder (C₂-C₁₈)-Alkenyl-Rest bedeutet, einschließlich der physiologisch wirksamen Salze, ausgenommen 3-Hydroxypyridin-2-carbonsäure-(glycylethylester)-amid.

6. Verbindungen der Formel I gemäß den Ansprüchen 1 bis 5, in denen
R¹, R² und R³ Wasserstoff,
A eine -CH₂-Gruppe,
B -CO₂G bedeutet, wobei
G einen linearen (C₁-C₁₈)-Alkyl-Rest bedeutet,
einschließlich der physiologisch wirksamen Salze, ausgenommen 3-Hydroxypyridin-2-carbonsäure-(glycylethylester)-amid,

7. Verfahren zur Herstellung von Verbindungen der Formel I gemäß den Ansprüchen 1 bis 6, in denen A eine -CH₂-Gruppe, in der ein Wasserstoff durch eine Methylgruppe ersetzt sein kann, und B CO₂G bedeuten, **dadurch gekennzeichnet, daß**
i1.) Pyridin-2-carbonsäuren der Formel II (R⁵ = H) mit den Aminoestern Formel III oder deren Salze zu den Amidestern der Formel I umgesetzt werden; oder
i2.) Pyridin-2-carbonsäureester der Formel II (R⁵ = niedrig Alkyl (= C₁-C₅)-Alkyl)) unter den Bedingungen der Aminolyse zu den Verbindungen der Formel I umgesetzt werden; oder 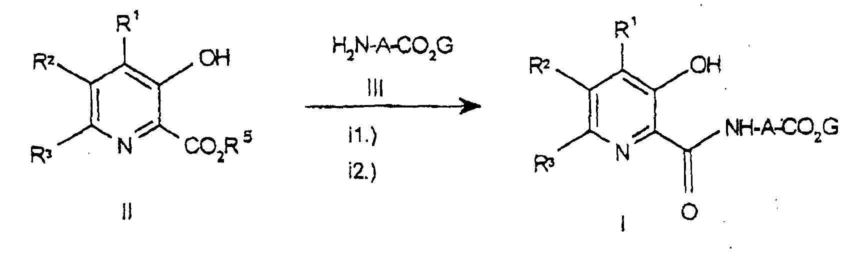
ii) die Verbindungen der Formel IV mit einem Alkohol GOH verestert werden; oder

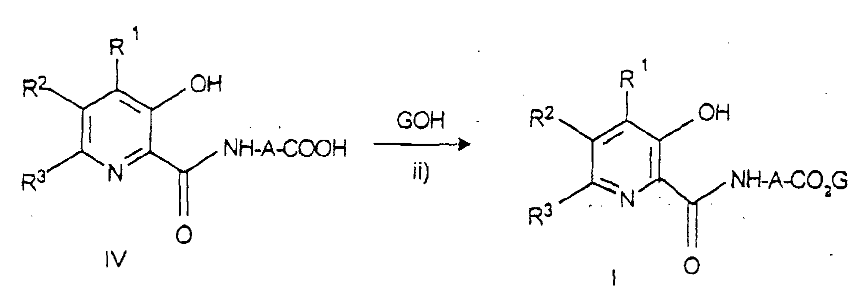

8. Verbindungen der Formel I gemäß den Ansprüchen 1 bis 6 zuzüglich 3-Hydroxypyridin-2-carbonsäure-(glycylethylester)amid zur Hemmung der Prolyl-4-hydroxylase in vivo.

9. Verbindungen der Formel gemäß den Ansprüchen 1 bis 6 zuzüglich 3-Hydroxypyridin-2-carbonsäure-(glycylethylester)amid zur Hemmung der Kollagenbiosynthese.

10. Verbindungen der Formel I gemäß den Ansprüchen 1 bis 6 zuzüglich 3-Hydroxypyridin-2-carbonsäure-(glycylethy)ester)amid zur Vermeidung/Verringerung der Ablagerungen von Bindegewebe (Kollagenen) in den erkrankten menschlichen Organen (Fibrose).

11. Verbindungen der Formel I gemäß den Ansprüchen 1 bis 6 zuzüglich 3-Hydroxypyridin-2-carbonsäure-(glycylethylester)amid zur Anwendung bei fibrotischen Erkrankungen.

12. Verbindungen der Formel I gemäß Anspruch 11 zuzüglich 3-Hydroxypyridin-2-carbonsäure-(glycylethylester)amid zur Anwendung bei fibrotischen Erkrankungen der Lunge, der Leber, der Niere, des Herzens, des Auges und der Haut und bei Artherosklerose.

13. Verbindungen der Formel I gemäß den Ansprüchen 11 und 12 zuzüglich 3-Hydroxypyridin-2-carbonsäure-(glycylethylester)amid wobei die Anwendung lokal und/oder systemisch erfolgt.

14. Verbindungen der Formel I gemäß Anspruch 13 zuzüglich 3-Hydroxypyridin-2-carbonsäure-(glycylethylester)amid zur lokalen Anwendung zur Vermeidung/Verringerung von Vernarbungen nach chirurgischen Eingriffen am menschlichen Körper.

15. Verbindungen der Formel I gemäß den Ansprüchen 8 bis 13 zuzüglich 3-Hydroxypyridin-2-carbonsäure-(glycylethy(ester)amid zur lokalen Anwendung bei erhöhter Bildung von Bindegewebe (Fibrose) in der Haut.

16. Verbindungen der Formel I gemäß den Ansprüchen 8 bis 13 zuzüglich 3-Hydroxypyridin-2-carbonsäure-(glycylethylester)amid zur lokalen Anwendung (inhalativ) bei erhöhter Bildung von Bindegewebe (Fibrose) in der Lunge.

17. Verbindungen der Formel I gemäß den Ansprüchen 8 bis 13 zuzüglich 3-Hydroxypyridin-2-carbonsäure-(glycylethylester)amid zur lokalen Anwendung bei erhöhter Bildung von Bindegewebe (Fibrose) am Auge.

18. Verbindungen der Formel I gemäß den Ansprüchen 8 bis 13 zuzüglich 3-Hydroxypyridin-2-carbonsäure-(glycylethylester)amid zur lokalen Anwendung am Auge zur nachoperativen Behandlung des Glaukoms.

19. Verbindungen der Formel I gemäß den Ansprüchen 8 bis 13 zuzüglich 3-Hydroxypyridin-2-carbonsäure-(glycylethylester)amid zur lokalen Anwendung bei strahleninduzierter oder durch Chemotherapie induzierter Fibrose, insbesondere Fibrosen der Lunge.

20. Arzneimittel, enthaltend eine oder mehrere Verbindungen der Formel I gemäß den Ansprüchen 1 bis 6 zuzüglich 3-Hydroxypyridin-2-carbonsäure-(glycylethylester)amid.

21. Verwendung der Verbindungen der Formel l gemäß den Ansprüchen 1 bis 6 zuzüglich 3-Hydroxypyridin-2-carbonsäure-(glycylethylester)amid zur Herstellung eines Arzneimittels zur Hemmung der Prolyl-4-hydroxylase und bei fibrotischen Erkrankungen.

## Claims

1. A compound of the formula I 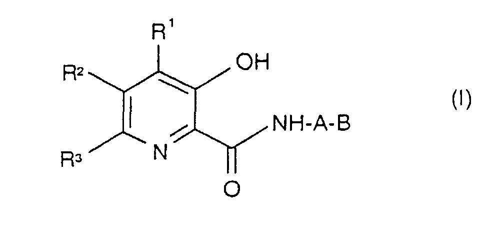 in which
R¹, R² and R³ are hydrogen,
A is a -CH₂- group, in which one hydrogen atom can be replaced by a methyl group, and
B is -CC₂G, where
G is a branched or unbranched (C₁-C₂₀)-alkyl radical, a branched or unbranched (C₂-C₂₀)-alkenyl radical, a corresponding (C₂-C₂₀)-alkynyl radical, a corresponding (C₄-C₂₀)-alkenynyl radical or a (C₃-C₁₃)-cycloalkyl radical, or a retinyl radical, where the radicals can in each case contain one or more multiple bonds, or is a phenylalkyl radical where the above radicals in particular contain one or more substituents from the group consisting of hydroxyl, halogen, cyano, trifluoromethyl, carboxyl, (C₁-C₁₂)-alkyl, (C₃-C₈)-cycloalkyl, (C₅-C₈)-cycloalkenyl, (C₁-C₁₂)-alkoxy, (C₁-C₁₂)-alkoxy-(C₁-C₁₂)-alkyl, (C₁-C₁₂)-alkoxy-(C₁-C₁₂)-alkoxy, phenyl-(C₁-C₄)-alkyloxy, (C₁-C₈)-hydroxyalkyl, (C₁-C₁₂)-alkylcarbonyloxy, (C₃-C₈)-cycloalkylcarbonyloxy, benzoyloxy, phenyl-(C₁-C₄)-alkylcarbonyloxy, including the physiologically active salts, excluding 3-hydroxypyridine-2-carboxylic acid (glycyl ethyl ester)amide.

2. A compound of the formula I as claimed in claim 1, in which
R¹, R² and R³ are hydrogen,
A is a -CH₂- group, in which one hydrogen atom can be replaced by a methyl group, and
B is -CO₂G, where
G is a branched or unbranched aliphatic (C₁-C₁₈)-alkyl radical, a (C₃-C₈)-cycloalkyl radical, a (C₃-C₈)-cycloalkyl-(C₁-C₈)-alkyl radical, a branched or unbranched (C₂-C₁₈)-alkenyl radical, such as, for example, a geranyl, farnesyl or a retinyl radical, or a corresponding (C₂-C₁₈)-alkynyl radical, a benzyl, phenethyl, phenylpropyl or phenylbutyl radical,
where the above radicals contain a substituent from the group consisting of hydroxyl, (C₁-C₄)-alkoxy, (C₁-C₆)-alkylcarbonyloxy, (C₃-C₈)-cycloalkylcarbonyloxy, benzoyloxy or phenyl-(C₁-C₄)-alkylcarbonyloxy, including the physiologically active salts, excluding 3-hydroxypyridine-2-carboxylic acid (glycyl ethyl ester)amide.

3. A compound of the formula I as claimed in claims 1 to 2, in which
R¹, R² and R³ are hydrogen,
A is a -CH₂- group, in which one hydrogen atom can be replaced by a methyl group, and
B is -CO₂G, where
G is a branched or unbranched aliphatic (C₁-C₁₈)-alkyl radical, a (C₅-C₆)-cycloalkyl radical, a (C₃-C₈)-cycloalkyl-(C₁-C₄)-alkyl radical, a branched or unbranched (C₂-C₁₈)-alkenyl radical, a benzyl, phenethyl, phenylpropyl or phenylbutyl radical, including the physiologically active salts, excluding 3-hydroxypyridine-2-carboxylic acid (glycyl) ethyl ester)amide.

4. A compound of the formula I as claimed in claims 1 to 3, in which
R¹, R² and R³ are hydrogen,
A is a -CH₂- group, in which one hydrogen atom can be replaced by a methyl group, and
B is -CO₂G, where
G is a branched or unbranched (C₁-C₁₈)-alkyl or (C₂-C₁₈)-alkenyl radical, including the physiologically active salts, excluding 3-hydroxypyridine-2-carboxylic acid (glycyl ethyl ester)amide.

5. A compound of the formula I as claimed in claims 1 to 4, in which
R¹, R² and R³ are hydrogen,
A is a -CH₂- group, and
B is -CO₂G, where
G is a branched or unbranched (C₁-C₁₈)-alkyl or (C₂-C₁₈)-alkenyl radical, including the physiologically active salts, excluding 3-hydroxypyridine-2-carboxylic acid (glycyl ethyl ester)amide.

6. A compound of the formula I as claimed in claims 1 to 5, in which
R¹, R² and R³ are hydrogen,
A is a -CH₂- group,
B is -CO₂G, where
G is a linear (C₁-C₁₈)-alkyl radical, including the physiologically active salts, excluding 3-hydroxypyridine-2-carboxylic acid (glycyl ethyl ester)amide.

7. A process for the preparation of compounds of the formula I as claimed in claims 1 to 6, wherein
A is a -CH₂- group, in which one hydrogen atom is replaced by a methyl group, and B is CO₂C, which comprises
i1.) reacting pyridine-2-carboxylic acids of the formula II (R⁵ = H) with the amino esters of the formula III or their salts to give the amidoesters of the formula I; or
i2.) reacting pyridine-2-carboxylic acid esters of the formula II (R⁵ = lower alkyl = (C₁-C₅)-alkyl) under the conditions of aminolysis to give the compounds of the formula I 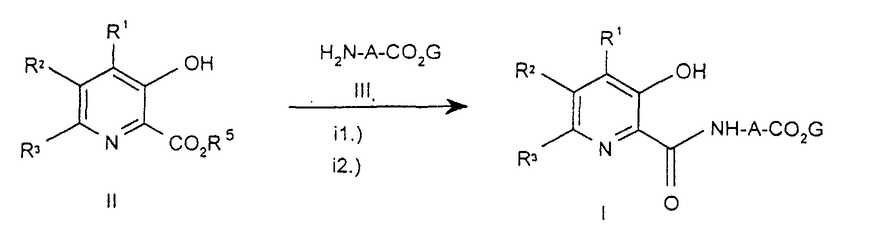
or
ii) esterifying the compounds of the formula IV with an alcohol GOH

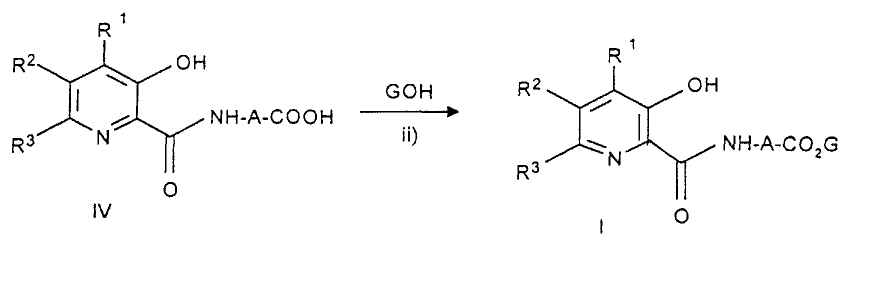

8. A compound of the formula I as claimed in claims 1 to 6 plus 3-hydroxypyridine-2-carboxylic acid (glycyl ethyl ester)amide for the inhibition of prolyl-4-hydroxylase in vivo.

9. A compound of the formula 1 as claimed in claims 1 to 6 plus 3-hydroxypyridine-2-carboxylic acid (glycyl ethyl ester)amide for the inhibition of collagen biosynthesis.

10. A compound of the formula I as claimed in claims 1 to 6 plus 3-hydroxypyridine-2-carboxylic acid (glycyl ethyl ester)amide for the avoidance/reduction of the deposits of connective tissue (collagens) in the diseased human organs (fibrosis).

11. A compound of the formula I as claimed in claim 1 to 6 plus 3-hydroxypyridine-2-carboxylic acid (glycyl ethyl ester)amide for use in fibrotic disorders.

12. A compound of the formula I as claimed in claim 11 plus 3-hydroxypyridine-2-carboxylic acid (glycyl ethyl ester)amide for use in fibrotic disorders of the lung, the liver, the kidney, the heart, the eye and the skin and in atherosclerosis.

13. A compound of the formula I as claimed in claims 11 and 12 plus 3-hydroxypyridine-2-carboxylic acid (glycyl ethyl ester)amide, where use takes place locally and/or systemically.

14. A compound of the formula I as claimed in claim 13 plus 3-hydroxypyridine-2-carboxylic acid (glycyl ethyl ester)amide for local use for the avoidance/reduction of scars after surgical interventions on the human body.

15. A compound of the formula I as claimed in claims 8 to 13 plus 3-hydroxypyridine-2-carboxylic acid (glycyl ethyl ester)amide for local use in the case of increased formation of connective tissue (fibrosis) in the skin.

16. A compound of the formula I as claimed in claims 8 to 13 plus 3-hydroxypyridine-2-carboxylic acid (glycyl ethyl ester)amide for local use (by inhalation) in the case of increased formation of connective tissue (fibrosis) in the lung.

17. A compound of the formula I as claimed in claims 8 to 13 plus 3-hydroxypyridine-2-carboxylic acid (glycyl ethyl ester)amide for local use in the case of increased formation of connective tissue (fibrosis) in the eye.

18. A compound of the formula I as claimed in claims 8 to 13 plus 3-hydroxypyridine-2-carboxylic acid (glycyl ethyl ester)amide for local use in the eye for postoperative treatment of glaucoma.

19. A compound of the formula I as claimed in claims 8 to 13 plus 3-hydroxypyridine-2-carboxylic acid (glycyl ethyl ester)amide for local use in radiation-induced fibrosis or fibrosis induced by chemotherapy, in particular fibroses of the lung.

20. A pharmaceutical comprising one or more compounds of the formula I as claimed in claims 1 to 6 plus 3-hydroxypyridine-2-carboxylic acid (glycyl ethyl ester)amide.

21. The use of the compounds of the formula I as claimed in claims 1 to 6 plus 3-hydroxypyridine-2-carboxylic acid (glycyl ethyl ester)amide for the production of a pharmaceutical for the inhibition of prolyl-4-hydroxylase and in fibrotic disorders.

## Revendications

1. Composés de formule générale I 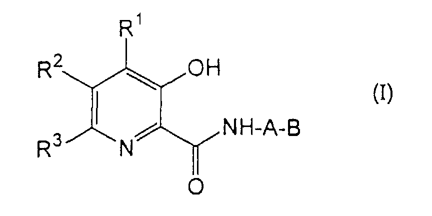 dans laquelle
R¹, R² et R³ sont l'hydrogène,
A est un groupe -CH₂ dans lequel un hydrogène peut être remplacé par un groupe méthyle, et
B est -CO₂G, où
G est un radical alkyle (en C₁-C₂₀) ramifié ou non ramifié, ou un radical alcényle (en C₂-C₂₀) ramifié ou non ramifié, un radical alcynyle (en C₂-C₂₀) correspondant, un radical alcéninyle (en C₄-C₂₀) correspondant ou un radical cycloalkyle (en C₃-C₁₂), ou un radical rétinyle, les radicaux pouvant dans chaque cas contenir une ou plusieurs liaisons multiples, ou un radical phénylalkyle, où les radicaux ci-dessus en particulier contiennent un ou plusieurs substituants de la série des groupe hydroxy, halogéno, cyano, trifluorométhyle, carboxy, alkyle (en C₁-C₁₂), cycloalkyle (en C₃-C₈), cycloalcényle (en C₅-C₈), alcoxy (en C₁-C₁₂), alcoxy (en C₁-C₁₂)-alkyle (en C₁-C₁₂), alcoxy (en C₁-C₁₂)-alcoxy (en C₁-C₁₂), phényl-alkyloxy (en C₁-C₄), hydroxyalkyle (en C₁-C₈), alkylcarbonyloxy (en C₁-C₁₂), cycloalkylcarbonyloxy (en C₃-C₈), benzoyloxy, phénylalkyl (en C₁-C₄)-carbonyloxy, y compris les sels actifs physiologiquement, à l'exception du (glycyléthylester)amide de l'acide 3-hydroxypyridin-2-carboxylique.

2. Composés de formule I, selon la revendication 1, dans lesquels
R¹, R² et R³ sont l'hydrogène,
A est un groupe -CH₂ dans lequel un hydrogène peut être remplacé par un groupe méthyle, et
B est -CO₂G, où
G est un radical alkyle (en C₁-C₁₈) aliphatique ramifié ou non ramifié, un radical cycloalkyle (en C₃-C₈), un radical cycloalkyl (en C₃-C₈)-alkyle (en C₁-C₈), un radical alcényle (en C₂-C₁₈) ramifié ou non ramifié, comme par exemple un radical géranyle, farnésyle ou rétinyle, ou un radical alcinyle (en C₂-C₁₈) correspondant, un radical benzyle, phénéthyle, phénylpropyle ou phénylbutyle,
où les radicaux ci-dessus contiennent un ou plusieurs substituants de la série des groupe hydroxy, alcoxy (en C₁-C₄), alkylcarbonyloxy (en C₁-C₆), cycloalkylcarbonyloxy (en C₃-C₈), benzoyloxy, ou phénylalkyl (en C₁-C₄)-carbonyloxy, y compris les sels actifs physiologiquement, à l'exception du (glycyléthylester)amide de l'acide 3-hydroxypyridin-2-carboxylique.

3. Composés de formule I, selon les revendications 1 à 2, dans lesquels
R¹, R² et R³ sont l'hydrogène,
A est un groupe -CH₂, dans lequel un hydrogène peut être remplacé par un groupe méthyle, et
B est -CO₂G, où
G est un radical alkyle (en C₁-C₁₈) aliphatique ramifié ou non ramifié, un radical cycloalkyle (en C₅-C₆), un radical cycloalkyl (en C₃-C₈)-alkyle (en C₁-C₄), un radical alcényle (en C₂-C₁₈) ramifié ou non ramifié, un radical benzyle, phénéthyle, phénylpropyle ou phénylbutyle, y compris les sels actifs physiologiquement, à l'exception du (glycyléthylester)amide de l'acide 3-hydroxypyridin-2-carboxylique.

4. Composés de formule I, selon les revendications 1 à 3, dans lesquels
R¹, R² et R³ sont l'hydrogène,
A est un groupe -CH₂ dans lequel un hydrogène peut être remplacé par un groupe méthyle, et
B est -CO₂G, où
G est un radical alkyle (en C₁-C₁₈) ou alcényle (en C₂-C₁₈) ramifié ou non ramifié, y compris les sels actifs physiologiquement, à l'exception du (glycyléthylester)amide de l'acide 3-hydroxypyridin-2-carboxylique.

5. Composés de formule I, selon les revendications 1 à 4, dans lesquels
R¹, R² et R³ sont l'hydrogène,
A est un groupe -CH₂, et
B est -CO₂G, où
G est un radical alkyle (en C₁-C₁₈) ou alcényle (en C₂-C₁₈) ramifié ou non ramifié, y compris les sels actifs physiologiquement, à l'exception du (glycyléthylester)amide de l'acide 3-hydroxypyridin-2-carboxylique.

6. composés de formule I, selon les revendications 1 à 5, dans lesquels
R¹, R² et R³ sont l'hydrogène,
A est un groupe -CH₂, et
B est -CO₂G, où
G est un radical alkyle (en C₁-C₁₈) linéaire,
y compris les sels actifs physiologiquement, à l'exception du (glycyléthylester)amide de l'acide 3-hydroxypyridin-2-carboxylique.

7. Procédé de préparation des composés de formule I, selon les revendications 1 à 6, dans lesquels A est un groupe -CH₂, dans lequel un hydrogène peut être remplacé par un groupe méthyle et B est CO₂G, **caractérisé en ce que**
i1.) on fait réagir les acides pyridin-2-carboxyliques de formule Il (R⁵ = H) avec des aminoesters de formule III ou leurs sels pour obtenir les amidoesters de formule I ; ou
i2.) on fait réagir les esters des acides pyridin-2-carboxyliques de formule II (R⁵ = alkyle inférieur (= alkyle en C₁-C₅) dans des conditions d'aminolyse pour obtenir les composés de formule I ; ou 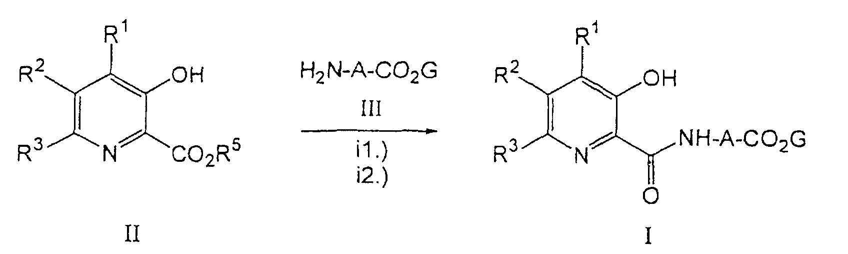
ii) on estérifie les composés de formule IV avec un alcool GOH ; ou

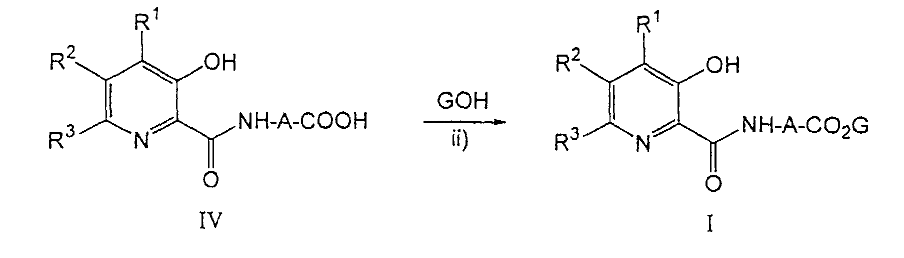

8. Composés de formule I, selon les revendications 1 à 6, en plus (glycyléthylester)amide de l'acide 3-hydroxypyridin-2-carboxylique, pour l'inhibition de la prolyl-4-hydroxyalse in vivo.

9. Composés de formule I, selon les revendications 1 à 6, en plus (glycyléthylester)amide de l'acide 3-hydroxypyridin-2-carboxylique, pour l'inhibition de la biosynthèse du collagène.

10. Composés de formule I, selon les revendications 1 à 6, en plus (glycyléthylester)amide de l'acide 3-hydroxypyridin-2-carboxylique, pour éviter/diminuer les formations de tissus conjonctifs (collagènes) dans les organes humains malades (fibroses).

11. Composés de formule I, selon les revendications 1 à 6, en plus (glycyléthylester)amide de l'acide 3-hydroxypyridin-2-carboxylique, pour l'utilisation pour des maladies fibrotiques.

12. Composés de formule I, selon la revendication 11, en plus (glycyléthylester)amide de l'acide 3-hydroxypyridin-2-carboxylique, pour l'utilisation pour des maladies fibrotiques des poumons, du foie, des reins, du coeur, de l'oeil et de la peau et pour l'athérosclérose.

13. Composés de formule I, selon les revendications 11 et 12, en plus (glycyléthylester)amide de l'acide 3-hydroxypyridin-2-carboxylique, dont l'utilisation se fait localement ou de manière systémique.

14. Composés de formule I, selon le revendication 13, en plus (glycyléthylester)amide de l'acide 3-hydroxypyridin-2-carboxylique, pour l'utilisation locale pour éviter/diminuer les cicatrices après des interventions chirurgicales sur le corps humain.

15. Composés de formule I, selon les revendications 8 à 13, en plus (glycyléthylester)amide de l'acide 3-hydroxypyridin-2-carboxylique, pour l'utilisation locale lors d'une formation augmentée du tissu conjonctif (fibrose) de la peau.

16. Composés de formule I, selon les revendications 8 à 13, en plus (glycyléthylester)amide de l'acide 3-hydroxypyridin-2-carboxylique, pour l'utilisation locale (par inhalation) lors d'une formation augmentée du tissu conjonctif (fibrose) dans les poumons.

17. Composés de formule I, selon les revendications 8 à 13, en plus (glycyléthylester)amide de l'acide 3-hydroxypyridin-2-carboxylique, pour l'utilisation locale lors d'une formation augmentée du tissu conjonctif (fibrose) sur l'oeil.

18. Composés de formule I, selon les revendications 8 à 13 en plus (glycyléthylester)amide de l'acide 3-hydroxypyridin-2-carboxylique, pour l'utilisation locale sur l'oeil pour le traitement postopératoire du glaucome.

19. Composés de formule I, selon les revendications 8 à 13, en plus (glycyléthylester)amide de l'acide 3-hydroxypyridin-2-carboxylique, pour l'utilisation locale lors d'une fibrose induite par rayons ou induite par chimiothérapie, en particulier les fibroses des poumons.

20. Médicament contenant un ou plusieurs composés de formule I, selon les revendications 1 à 6, en plus (glycyléthylester)amide de l'acide 3-hydroxypyridin-2-carboxylique.

21. Utilisation de composés de formule I selon les revendications 1 à 6, en plus (glycyléthylester)amide de l'acide 3-hydroxypyridin-2-carboxylique pour la fabrication d'un médicament pour l'inhibition de la propyl-4-hydroxylase et pour des maladies fibrotiques.
